# EUROPÄISCHE PATENTSCHRIFT

(11) **EP 1 411 211 B1**
(45) Veröffentlichungstag und Bekanntmachung des Hinweises auf die Patenterteilung: **10.01.2007**
(21) Anmeldenummer: 02102458.3
(22) Anmeldetag: 17.10.2002
(51) Int. Cl.: F01L 1/46, A61F 2/08, F15B 15/10, B25J 9/14, F01L 13/00

(54) **Ventileinrichtung für ein Kraftfahrzeug**
Valve system for a motor vehicle
Dispositif de soupape pour un véhicule automobile

(43) Veröffentlichungstag der Anmeldung: 21.04.2004
(73) Patentinhaber: Ford Global Technologies, LLC., Dearborn, MI 48126 (US)
(72) Erfinder: Kluge, Torsten, 51491, Overath (DE)
(74) Vertreter: Drömer, Hans-Carsten

(56) Entgegenhaltungen:
- EP-A- 0 143 128
- EP-A- 0 924 033
- WO-A-02/22492
- DE-A- 10 017 104
- DE-C- 4 027 630
- FR-A- 2 821 387
- US-A- 5 099 884
- US-A- 6 109 852

## Beschreibung

Die Erfindung betrifft eine Ventileinrichtung mit einem beweglichen Ventilelement zur Steuerung des Gasflusses in einer Brennkraftmaschine.

Ventileinrichtungen werden in verschiedenen Bereichen der Technik zur Steuerung des Flusses eines gasförmigen oder flüssigen Mediums benötigt. Nachfolgend sollen in Brennkraftmaschinen von Kraftfahrzeugen verwendete Ventileinrichtungen betrachtet werden.

Ventileinrichtungen von Brennkraftmaschinen dienen dazu, den Einlaß der Verbrennungsluft beziehungsweise den Auslaß der Abgase zu steuern. Typischerweise wird dabei ein bewegliches Ventilelement in Form eines Ventilstößels linear zwischen einer Offenstellung und einer Schließstellung hin und her bewegt. Die erforderliche Kraft zur Ausführung dieser Bewegung kann gemäß dem Stand der Technik z. B. durch Nockenwellen oder mittels elektromagnetischer Spulen bzw. hydraulischer oder pneumatischer Aktuatoren erzeugt werden. Bei Verwendung bekannter Aktuatoren besteht jedoch hinsichtlich der Kompaktheit und der Leistungsfähigkeit noch ein Verbesserungsbedarf. Ein wichtiges Ziel dieser Verbesserungen besteht darin, bei Verwendung idealer Ventile die Leistung einer Brennkraftmaschine ohne Einsatz einer Drosselklappe allein durch die Zeitsteuerung bzw. das Timing der Ventilöffnung und durch den Ventilhub regeln zu können.

Aus EP 0 143 128 ist eine Ventilbetätigungsvorrichtung für einen Verbrennungsmotor oder eine ähnliche, Ventile aufweisende Maschine bekannt, welche eine piezoelektrische Betätigungseinheit aufweist, die das Motorventil entsprechend der Ausdehnung der Betätigungseinheit antreibt, wobei Steuermittel die elektrischen Eingangsgrößen der piezoelektrischen Betätigungseinheit entsprechend zugeführten Motorparametern beeinflussen.

Aus WO 02/22492 A2 ist ein durch elektrostatische Kräfte angetriebenes MEMS-Ventil bekannt, wobei eine Membran aus mehreren Schichten, die z.B. ein Polymermaterial aufweisen können, durch Anlegen einer Spannung relativ zu einer Ventilöffnung bewegbar ist, um den von der Membran abgedeckten Abschnitt der Ventilöffnung einzustellen.

Es ist eine Aufgabe der vorliegenden Erfindung, eine für den Einsatz in Brennkraftmaschinen geeignete Ventileinrichtung bereitzustellen, die eine leistungsfähige, flexible und hitzestabile Ventilsteuerung ermöglicht.

Diese Aufgabe wird durch eine Ventileinrichtung mit den Merkmalen des Anspruchs 1 gelöst.

Vorteilhafte Ausgestaltungen sind in den Unteransprüchen enthalten.

Die erfindungsgemäße Ventileinrichtung mit einem beweglichen Ventilelement ist dadurch gekennzeichnet, daß diese wenigstens ein mit dem Ventilelement gekoppeltes künstliches Muskelelement aufweist, welches Kohlenstoff-Nanoröhrchen als aktive Elemente aufweist. Dabei kann der Terminus "Kopplung" auch eine physische Identität von Ventilelement und Muskelelement bedeuten.

Bei künstlichen Muskelelementen handelt es sich um neuartige Aktuatoren, welche in ihren Eigenschaften der natürlichen Muskulatur ähneln bzw. nachgebildet sind. Charakteristisch für künstliche Muskeln ist insbesondere eine im Volumen stattfindende Krafterzeugung aufgrund atomarer oder molekularer Wechselwirkungen. Häufig bestehen künstliche Muskeln - ähnlich wie natürliche Muskeln - aus einem formveränderlichen, weichen Material. Die Krafterzeugung in bekannten künstlichen Muskeln kann z. B. auf elektrostatischen Anziehungskräften, auf dem piezoelektrischen Effekt, auf einer Ultraschallerzeugung, auf einem Formgedächtnis von Materialien, auf einem Ionenaustausch, auf einer Streckung von Kohlenstoff-Nanoröhrchen und/oder auf der Einlagerung von Wasserstoff in Metallhydride beruhen. Je nach Wirkungsprinzip können künstliche Muskeln aus Polymeren, insbesondere Polymer-Gelen, aus ferroelektrischen Substanzen, aus Silizium, aus Legierungen mit einem Formgedächtnis od. dgl. hergestellt sein. Eine detaillierte Beschreibung verschiedener Arten künstlicher Muskeln ist z. B. in der EP 0 924 033 A2, der US 2002/0026794 A1, der US 6 109 852 und ähnlicher Patentliteratur zu finden. Darüber hinaus sind Beispiele künstlicher Muskeln in Publikationen der einschlägigen Forschungsinstitute beschrieben (z. B. Max-Planck-Institut für Festkörperforschung in Stuttgart; Abteilung für künstliche Intelligenz des MIT, Massachusetts, USA).

In der beschriebenen Ventileinrichtung finden künstliche Muskelelemente Einsatz, die durch ein elektrisches Signal gesteuert werden können. Insbesondere kann dabei die vom Muskelelement erzeugte mechanische Energie aus der elektrischen Energie des Signals stammen. Elektrisch gesteuerte künstliche Muskelelemente haben den Vorteil, daß diese mit der üblichen Steuerungstechnik einer Brennkraftmaschine kompatibel sind.

In der Ventileinrichtung werden vorzugsweise derartige künstliche Muskelelemente eingesetzt, welche aktiv kontrahieren, aktiv expandieren und/oder aktiv ihre Form - wie z. B. ihre Krümmung - verändern können. Künstliche Muskelelemente, die in zwei entgegengesetzte Richtungen eine Kraft aktiv erzeugen können, können dabei bereits einzeln die gesamte Bewegung des Ventilelementes veranlassen. Wenn ein künstliches Muskelelement dagegen eine aktive Kraft nur in einer Richtung erzeugen kann, ist es durch einen in der Gegenrichtung wirksamen Krafterzeuger, z. B. ein weiteres künstliches Muskelelement, zu ergänzen, um das Ventilelement in Öff nungs- und Schließrichtung steuerbar zu machen.

Erfindungsgemäß werden künstliche Muskelelemente verwendet, welche auf der Wechselwirkung von Kohlenstoff-Nanoröhrchen basieren. Derartige künstliche Muskelelemente zeichnen sich durch eine hohe Hitzebeständigkeit bis zu 1000°C aus. Ferner können derartige Muskelelemente durch elektrische Energie gesteuert und expandiert werden (vgl. Science vom 21.05.1999). Ein weiterer bevorzugter Typ eines künstlichen Muskels beruht auf Polymer-Gelen (vgl. Low, L. W.; Madou, M. J. "Microactuators towards microvalves for controlled drug delivery", Sensors and Actuators B: Chemical, 67 (1-2) (2000) pp. 149-160).

Für die konstruktive Ausgestaltung des Ventilelementes gibt es zahlreiche Möglichkeiten. Um die Änderungen an bestehenden Motoren gering zu halten, werden dabei vorzugsweise möglichst viele bekannte Konstruktionselemente übernommen. Insbesondere kann das Ventilelement durch einen verschiebebeweglich gelagerten Ventilstößel gebildet werden, mit welchem das künstliche Muskelelement direkt oder indirekt, d. h. über Zwischenbauteile wie z. B. Kipp- oder Schlepphebel, gekoppelt ist.

Gemäß einer speziellen Ausgestaltung der vorstehend genannten Ventileinrichtung mit einem Ventilstößel ist Letzterer mit einem Vorspannelement gekoppelt, z. B. mit einer Schraubenfeder. Das Vorspannelement kann dabei eine Kraft in eine Bewegungsrichtung des Ventilstößels erzeugen. Das künstliche Muskelelement muß dann nur noch eine Kraft in die Gegenrichtung erzeugen können, um das Ventilelement insgesamt nach Belieben steuerbar zu machen.

Bei einer anderen Ausgestaltung der Ventileinrichtung mit einem Ventilstößel ist Letzterer mit einer Gasdruckkammer derart gekoppelt, daß eine Druckerhöhung in der Gasdruckkammer eine Bewegung des Ventilstößels bewirkt. Durch den Druck in der Gasdruckkammer kann daher aktiv eine Kraft auf den Ventilstößel ausgeübt werden.

Vorzugsweise werden bei der letztgenannten Ausgestaltung mit einer Gasdruckkammer die Wandungen der Gasdruckkammer ganz oder zumindest teilweise von dem künstlichen Muskelelement gebildet. Diese Doppelfunktion des künstlichen Muskelelementes trägt einerseits zu einer kompakteren und Material sparenden Konstruktion bei und erlaubt andererseits eine unmittelbare Wechselwirkung zwischen dem künstlichen Muskelelement und dem Druck in der Gasdruckkammer.

Bei einer anderen Weiterbildung der Ventileinrichtung wird das Ventilelement durch eine schwenkbewegliche Klappe gebildet, welche je nach Einstellwinkel eine Durchflussöffnung freigeben beziehungsweise verschließen kann. Insbesondere können dabei auch mehrere derartige Klappen zusammenwirken, um gemeinsam die Durchflussöffnung zu kontrollieren. Ein derartiges Ventilelement mit mehreren Klappen ist einerseits robuster gegen einen Totalausfall und kann andererseits schneller betätigt werden, da mehrere Klappen gleichzeitig einen Weg in die Öffnungs- bzw. Schließstellung zurücklegen.

Ferner sind auch Ausgestaltungen der Ventileinrichtung möglich, bei denen das Ventilelement durch das künstliche Muskelelement selbst gebildet wird, d.h. hiermit physisch identisch ist. Dabei kann das künstliche Muskelelement z. B. in einem Durchlaß angeordnet sein und diesen durch eine aktive Form- oder Volumenveränderung verschließen oder freigeben.

Im Folgenden wird die Erfindung mit Hilfe der Figuren beispielhaft näher erläutert. Es zeigen:
- Fig. 1: einen Schnitt durch eine erste Ausgestaltung einer erfindungsgemäßen Ventileinrichtung, bei welcher künstliche Muskeln als Protagonisten und ein Gasdruck als Antagonist wirken;
- Fig. 2: einen Schnitt durch eine zweite Ausgestaltung einer erfindungsgemäßen Ventileinrichtung, bei welcher unterschiedliche künstliche Muskeln als Antagonisten und Protagonisten wirken;
- Fig. 3: einen Schnitt durch eine dritte Ausgestaltung einer erfindungsgemäßen Ventileinrichtung, bei welcher künstliche Muskeln und eine Schraubenfeder mittelbar am oberen Ende eines Ventilstößels angreifen;
- Fig. 4: einen Schnitt durch eine vierte Ausgestaltung einer erfindungsgemäßen Ventileinrichtung, bei welcher künstliche Muskeln an Schlepphebeln angreifen;
- Fig. 5: einen Schnitt durch eine fünfte Ausgestaltung einer erfindungsgemäßen Ventileinrichtung, bei welcher schwenkbewegliche Klappen von künstlichen Muskeln gesteuert werden;
- Fig. 6: die Anordnung von Klappenventilen gemäß Figur 5 im Kopf eines Motorzylinders, und
- Fig. 7: die Anordnung von ringförmig schließenden künstlichen Muskelelementen im Kopf eines Motorzylinders.

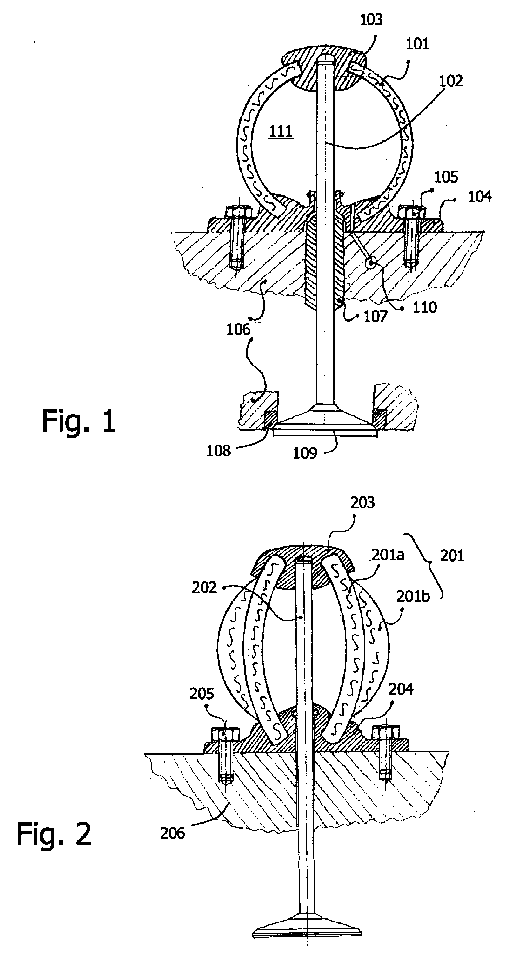

Figur 1 zeigt in einer Schnittdarstellung eine erste Ausführungsform einer erfindungsgemäßen Ventileinrichtung, welche insbesondere als Einlaß- oder Auslaßventil am Zylinderkopf 106 einer Brennkraftmaschine eingesetzt werden kann. Entsprechend der an sich bekannten Bauweise derartiger Ventileinrichtungen weist diese einen linear (auf und ab) verschiebebeweglichen Ventilstößel 102 auf, an dessen unterem Ende ein Ventilteller 109 angeordnet ist. Der Ventilteller 109 wirkt mit einem im Zylinderkopf 106 ausgebildeten Ventilsitz 108 zusammen, um eine Öffnung für einen Gasfluß wahlweise freizugeben oder zu schließen. Zur Abdichtung des Ventilelementes nach außen und zu dessen möglichst reibungsarmer Führung ist im Durchlaß des Ventilstößels 102 durch den Zylinderkopf 106 eine Ventilführung 107 vorgesehen.

Neuartig an dem in Figur 1 dargestellten Ventilelement ist der Aktuator zur Erzeugung einer aktiven Kraft auf den Ventilstößel 102. Dieser Aktuator wird durch künstliche Muskelelemente 101 gebildet, welche einerseits an einer mit Schrauben 105 auf dem Zylinderkopf 106 befestigten Basisplatte 104 und andererseits an einer Kopfplatte 103 befestigt sind. In der Kopfplatte 103, die wie die Basisplatte 104 typischerweise aus Metall besteht, sitzt ferner das obere Ende des Ventilstößels 102.

Bei den künstlichen Muskelelementen 101 kann es sich vorzugsweise um solche auf der Basis von Kohlenstoff-Nanoröhrchen handeln, welche besonders hitzebeständig sind und welche sich durch elektrische Signale gesteuert expandieren können (vgl. Science vom 21.05.1999). Ebenso sind jedoch auch künstliche Muskeln auf der Basis von Polymer-Hydrogelen einsetzbar, die durch elektrische Signale gesteuert kontrahieren können, oder künstliche Muskeln, die sowohl eine aktive Kontraktion als auch Expansion erlauben. Ferner sind künstliche Muskeln mit einem geringen Verhältnis von Expansion zu Kontraktion geeignet (z.B. Kohlenstoff-Nanoröhrchen), welche in Papier-ähnlichen Mehrschichtstrukturen gebündelt sind und eine erhebliche Krümmung der gesamten Muskelstruktur erlauben. Eine nach unten gerichtete Bewegung des Ventilstößels 102 und damit ein Öffnen des Ventils kann durch eine aktive Kontraktion beziehungsweise Biegung der künstlichen Muskelelemente 101 bewirkt werden. Falls die künstlichen Muskelelemente 101 aktiv expandieren bzw. sich strecken können, können diese auch die entgegengesetzte Schließbewegung des Ventilstößels 102 bewirken.

Zusätzlich oder alternativ kann eine Schließbewegung des Ventilstößels 102 mit Hilfe einer Gasdruckkammer 111 bewirkt werden, welche das obere, außerhalb des Zylinderkopfes 106 befindliche Ende des Ventilstößels 102 umgibt und welche durch die künstlichen Muskelelemente 101, die Kopfplatte 103 sowie die Basisplatte 104 begrenzt wird. Wenn die genannten Elemente eine geschlossene Hülle bilden und die künstlichen Muskeln 101 z. B. durch eine entsprechende Beschichtung gasdicht ausgebildet sind, kann in der Gasdruckkammer 111 wahlweise ein höherer Druck erzeugt werden, der zu einer Aufwärtsbewegung des Ventilstößels 102 in die Schließstellung führt. Diese Wirkung der Gasdruckkammer kann ggf. mit einer aktiven Expansion der künstlichen Muskelelemente 101 kombiniert werden, falls Letztere dazu in der Lage sind. Für die Erzeugung eines höheren Druckes in der Gasdruckkammer 111 beziehungsweise für einen Druckabbau ist die Gasdruckkammer 111 über einen Kanal 110 mit einer geregelten Gasdruckquelle (nicht dargestellt) verbunden.

Figur 2 zeigt eine abgewandelte Ausführungsform einer Ventileinrichtung, wobei ähnliche oder identische Teile wie bei Figur 1 hier sowie bei den übrigen Figuren mit in den letzten beiden Ziffern identischen Bezugszeichen versehen sind.

Die Ventileinrichtung von Figur 2 besteht aus einem auf und ab beweglichen Ventilstößel 202, der durch einen Zylinderkopf 206 geführt und an seinem oberen Ende in einer Kopfplatte 203 befestigt ist. Weiterhin sind als Antagonisten wirkende erste künstliche Muskelelemente 201a an der Kopfplatte 203 sowie an einer Basisplatte 204, die am Zylinderkopf 206 verschraubt ist, befestigt.

Die Gegenkraft zu den Antagonisten 201a wird durch als Protagonisten wirkende künstliche Muskelelemente 201 b erzeugt, welche ebenfalls an der Kopfplatte 203 sowie an der Basisplatte 204 befestigt sind. Im Unterschied zu den Antagonisten 201 a können die Protagonisten 201 b eine aktive Expansion beziehungsweise Streckung ausführen und hierdurch eine nach oben gerichtete Kraft auf den Ventilstößel 202 erzeugen. Falls die von den Antagonisten 201a und den Protagonisten 201 b gebildete Wandung gasdicht ist, kann sie wie bei Figur 1 als Gasdruckkammer wirken. Weiterhin kann die Aktivierung und Deaktivierung von Antagonisten 201a und Protagonisten 201b jeweils reziprok erfolgen.

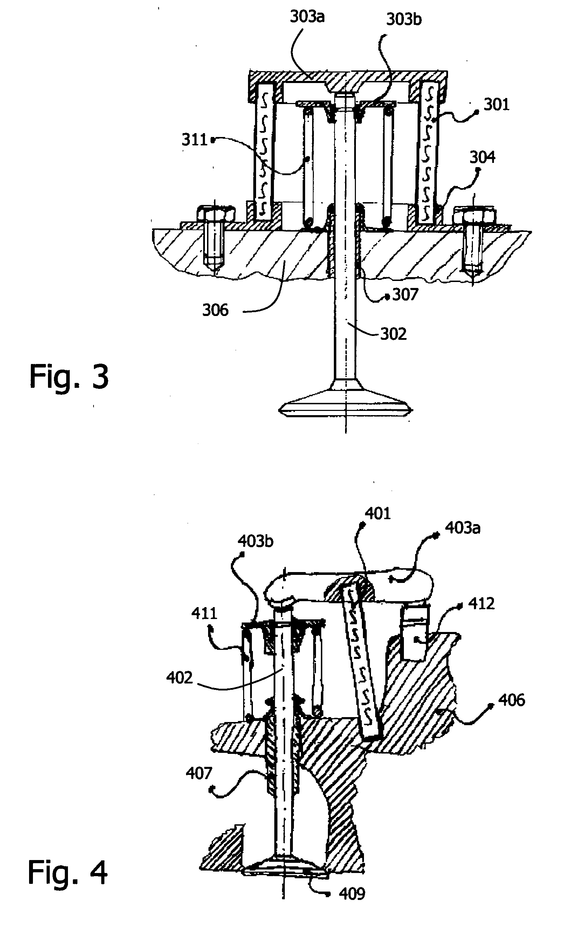

Figur 3 zeigt eine Ventileinrichtung, bei welcher der durch den Zylinderkopf 306 geführte Ventilstößel 302 an seinem oberen Ende einen Ventilfederteller 303b trägt. Zwischen dem Ventilfederteller und dem Zylinderkopf 306 stützt sich eine Ventilfeder 311 ab, welche eine nach oben gerichtete Vorspannung auf den Ventilstößel 302 ausübt und diesen daher in die Schließstellung drängt. Um den Ventilstößel 302 nach unten in eine Offenstellung bewegen zu können, sind künstliche Muskelelemente 301 vorgesehen, die sich zwischen einer Kopfplatte 303a und einer am Zylinderkopf 306 festgeschraubten Basisplatte 304 erstrecken und welche aktiv kontrahieren und/oder sich krümmen können. Da die Kopfplatte 303a sich auf dem oberen Ende des Ventilstößels 302 abstützt, wird Letzterer bei einer Kontraktion der künstlichen Muskeln 301 nach unten gedrückt. Die künstlichen Muskeln 301 verhindern bei dieser Anordnung ferner, daß die Kopfplatte 303a verloren geht.

Vorteilhaft bei einer derartigen Anordnung ist, daß die Schraubenfeder 311 eine Rückstellkraft aufbringt, so daß künstliche Muskeln 301 verwendet werden können, die nur in einer Richtung eine Kraft erzeugen können. Ferner können viele Merkmale herkömmlicher Ventileinrichtungen beibehalten werden.

Letzteres gilt auch für die in Figur 4 gezeigte Ventileinrichtung, bei welcher der Ventiistößei 402 über einen schwenkbeweglich gelagerten Schwinghebel 403a nach unten in eine Offenstellung gedrückt werden kann. Die in die Schließstellung drängende Rückstellkraft wird ähnlich wie bei Figur 3 durch eine Schraubenfeder 411 aufgebracht, welche sich zwischen dem Zylinderkopf 406 und einem Ventilfederteller 403b abstützt.

Das künstliche Muskelelement 401 ist wie in Figur 4 dargestellt oder in äquivalenter Weise zwischen dem Zylinderkopf 406 und dem Schwinghebel 403a angeordnet. Durch eine Kontraktion und/oder Biegung kann es eine nach unten gerichtete Kraft auf den Schwinghebel 403a ausüben, welche diesen nach unten zieht und den Ventilstößel 402 dadurch mittelbar in eine Offenstellung überführt. Weiterhin verhindert der künstliche Muskel 401, daß der Schwinghebel 403a verloren geht.

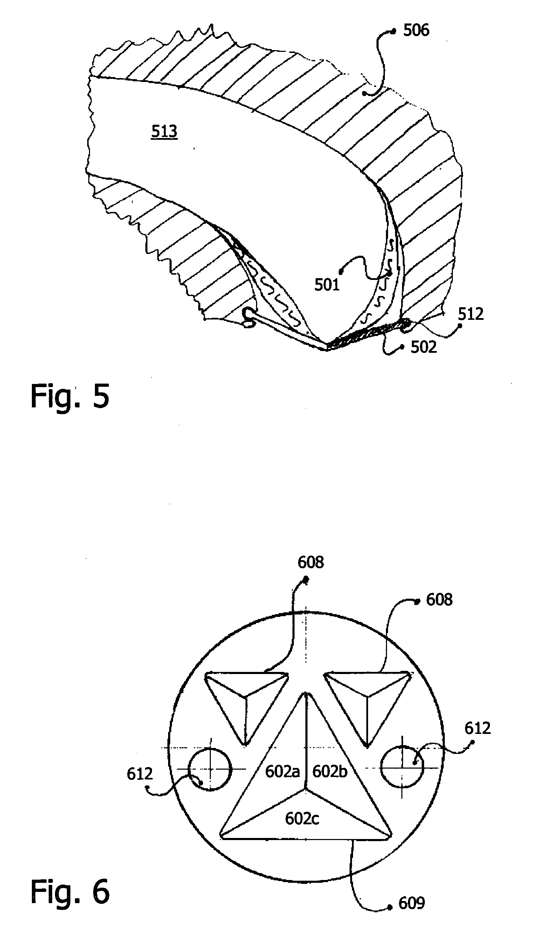

In den Figuren 5 und 6 ist eine Ventileinrichtung gezeigt, welche stärker von bekannten Konstruktionsprinzipien abweicht. Als Ventilelemente sind hierbei Klappen 502 vorgesehen, die am Ende eines Gaskanals 513 schwenkbeweglich in gasdichten Scharniergelenken 512 gelagert sind. An den Klappen 502 ist jeweils mindestens ein künstliches Muskelelement 501 befestigt, welches mit seinem anderen Ende am Zylinderkopf 506 ansetzt. Durch eine Kontraktion/Expansion bzw. Biegung/Streckung der künstlichen Muskelelemente 501 können die Ventilklappen 502 verschwenkt und daher aus der in Figur 5 gezeigten Schließstellung in eine Offenstellung überführt werden.

Figur 6 zeigt einen Zylinderkopf von unten. Dabei sind neben den zwei Zündkerzen 612 zwei dreieckige Auslaßöffnungen 608 sowie eine große dreieckige Einlaßöffnung 609 erkennbar. Die genannten Öffnungen werden jeweils durch drei dreieckige Ventilklappen 602a, 602b, 602c kontrolliert, die in Figur 6 im geschlossenen Zustand dargestellt sind, in welchem sie dichtend aneinander anliegen. Falls notwendig, z. B. bei Ventilöffnungen mit nur einer Klappe, könnten die Klappen auch dichtend auf dem Rand der Ventilöffnung anliegen.

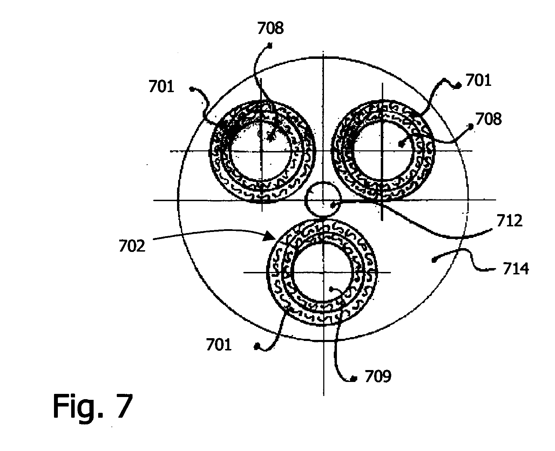

In Figur 7 ist eine weitere Ausgestaltung einer Ventileinrichtung in einer Ansicht wie bei Figur 6, d. h. von unten auf den Zylinderkopf, dargestellt. Zu erkennen sind eine zentral angeordnete Zündkerze 712 sowie zwei Einlaßöffnungen 708 und eine Auslaßöffnung 709. Am Rand der genannten Öffnungen sind künstliche Muskelelemente 701 ringförmig angeordnet. Jeder der künstlichen Muskeln 701 ist als flache Spirale ausgebildet, welche eine gasdichte Schichtstruktur bildet. Falls erforderlich, kann die Gasdichtheit durch eine Oberflächenbeschichtung sichergestellt werden. In Figur 7 sind die künstlichen Muskeln 701 in einer zurückgezogenen Öffnungsstellung dargestellt, in welcher sie einen Durchlaß durch die Ventilöffnungen freigeben. Bei entsprechender Ansteuerung können sich die künstlichen Muskeln 701 komplett zusammenziehen und hierdurch die zugehörige Ventilöffnung verschließen.

Die in den Figuren dargestellten Ventileinrichtungen ermöglichen eine flexible Steuerung des Ventils und z. B. eine verzögerte Ventilöffnung oder einen verringerten Ventilhub. Ferner können die Ventilelemente 102-702 wahlweise auch in jeder Zwischenstellung ihres Arbeitsbereiches gehalten werden.

## Patentansprüche

1. Ventileinrichtung zur Steuerung des Gasflusses in einer Brennkraftmaschine, enthaltend ein bewegliches Ventilelement (102-702) und wenigstens ein mit dem Ventilelement (102-702) gekoppeltes künstliches Muskelelement (101-701),
**dadurch gekennzeichnet, dass**
das künstliche Muskelelement (101-701) Kohlenstoff-Nanoröhrchen als aktive Elemente aufweist.

2. Ventileinrichtung nach Anspruch 1,
**dadurch gekennzeichnet, daß**
das künstliche Muskelelement (101-701) durch ein elektrisches Signal gesteuert werden kann.

3. Ventileinrichtung nach Anspruch 1 oder 2,
**dadurch gekennzeichnet, daß**
das künstliche Muskelelement (101-701) aktiv kontrahieren, expandieren und/oder seine Form verändern kann.

4. Ventileinrichtung nach mindestens einem der Ansprüche 1 bis 3,
**dadurch gekennzeichnet, daß**
das künstliche Muskelelement (101-701) ferner Polymer-Gele als aktive Elemente aufweist.

5. Ventileinrichtung nach mindestens einem der Ansprüche 1 bis 4,
**dadurch gekennzeichnet, daß** das Ventilelement ein verschiebebeweglich gelagerter Ventilstößel (102-402) ist, und daß das künstliche Muskelelement (101-401) direkt oder indirekt hiermit gekoppelt ist.

6. Ventileinrichtung nach Anspruch 5,
**dadurch gekennzeichnet, daß**
der Ventilstößel (302-402) mit einem Vorspannelement (311-411) gekoppelt ist, welches eine Kraft in eine Bewegungsrichtung des Ventilstößels erzeugt.

7. Ventileinrichtung nach Anspruch 5 oder 6,
**dadurch gekennzeichnet, daß**
der Ventilstößel (102) mit einer Gasdruckkammer (111) derart gekoppelt ist, daß eine Druckbeaufschlagung der Gasdruckkammer eine Bewegung des Ventilstößels bewirkt.

8. Ventileinrichtung nach Anspruch 7,
**dadurch gekennzeichnet, daß**
die Wandungen der Gasdruckkammer (111) ganz oder teilweise von dem künstlichen Muskelelement (101) gebildet werden.

9. Ventileinrichtung nach mindestens einem der Ansprüche 1 bis 8,
**dadurch gekennzeichnet, daß**
das Ventilelement als schwenkbeweglich gelagerte Klappe (502, 602) ausgebildet ist.

10. Ventileinrichtung nach mindestens einem der Ansprüche 1 bis 9,
**dadurch gekennzeichnet, daß**
das Ventilelement (702) durch das künstliche Muskelelement (701) gebildet wird.

## Claims

1. Valve device for controlling the gas flow in an internal combustion engine, containing a movable valve element (102-702), and at least one artificial muscle element (101-701) coupled to the valve element (102-702), **characterized in that** the artificial muscle element (101-701) contains carbon nonotubes as active elements.

2. Valve device according to Claim 1, **characterized in that** the artificial muscle element (101-701) can be controlled by means of an electrical signal.

3. Valve device according to Claim 1 or 2,
**characterized in that** the artificial muscle element (101-701) can actively contract, expand and/or change its form.

4. Valve device according to at least one of Claims 1 to 3, **characterized in that** the artificial muscle element (101-701) futhermore contains polymer gels as active elements.

5. Valve device according to at least one of Claims 1 to 4, **characterized in that** the valve element is a valve tappet (102-402) mounted with displacement movability, and **in that** the artificial muscle element (101-401) is coupled directly or indirectly to the latter.

6. Valve device according to Claim 5, **characterized in that** the valve tappet (302-402) is coupled to a prestressing element (311-411) which generates a force in one direction of movement of the valve tappet.

7. Valve device according to Claim 5 or 6, **characterized in that** the valve tappet (102) is coupled to a gas pressure chamber (111) in such a way that the action of pressure upon the gas pressure chamber causes a movement of the valve tappet.

8. Valve device according to Claim 7, **characterized in that** the walls of the gas pressure chamber (111) are formed completely or partially by the artificial muscle element (101).

9. Valve device according to at least one of Claims 1 to 8, **characterized in that** the valve element is designed as a pivotally movably mounted flap (502, 602).

10. Valve device according to at least one of Claims 1 to 9, **characterized in that** the valve element (702) is formed by the artificial muscle element (701).

## Revendications

1. Dispositif de soupape pour commander le flux de gaz dans un moteur à combustion interne, contenant un élément de soupape mobile (102-702) et au moins un élément de muscle artificiel (101-701) accouplé à l'élément de soupape (102-702),
**caractérisé en ce que**
l'élément de muscle artificiel (101-701) présente des nanotubes en carbone en tant qu'éléments actifs.

2. Dispositif de soupape selon la revendication 1,
**caractérisé en ce que**
l'élément de muscle artificiel (101-701) peut être commandé par un signal électrique.

3. Dispositif de soupape selon la revendication 1 ou 2,
**caractérisé en ce que**
l'élément de muscle artificiel (101-701) peut se contracter, se dilater et/ou modifier sa forme activement.

4. Dispositif de soupape selon au moins l'une quelconque des revendications 1 à 3,
**caractérisé en ce que**
l'élément de muscle artificiel (101-701) présente en outre des gels polymères en tant qu'éléments actifs.

5. Dispositif de soupape selon au moins l'une quelconque des revendications 1 à 4,
**caractérisé en ce que**
l'élément de soupape est un poussoir de soupape (102-402) monté de manière mobile en coulissement, et **en ce que** l'élément de muscle artificiel (101-401) lui est accouplé directement ou indirectement.

6. Dispositif de soupape selon la revendication 5,
**caractérisé en ce que**
le poussoir de soupape (302-402) est accouplé à un élément de précontrainte (311-411) qui produit une force dans une direction de déplacement du poussoir de soupape.

7. Dispositif de soupape selon la revendication 5 ou 6,
**caractérisé en ce que**
le poussoir de soupape (102) est accouplé à une chambre de pression à gaz (111) de telle sorte qu'une sollicitation par pression de la chambre de pression à gaz provoque un déplacement du poussoir de soupape.

8. Dispositif de soupape selon la revendication 7,
**caractérisé en ce que**
les parois de la chambre de pression à gaz (111) sont formées complètement ou partiellement par l'élément de muscle artificiel (101).

9. Dispositif de soupape selon au moins l'une quelconque des revendications 1 à 8,
**caractérisé en ce que**
l'élément de soupape est réalisé sous la forme d'un volet (502, 602) monté mobile à pivotement.

10. Dispositif de soupape selon au moins l'une quelconque des revendications 1 à 9,
**caractérisé en ce que**
l'élément de soupape (702) est formé par l'élément de muscle artificiel (701).
